# FASCICULE DE BREVET EUROPEEN

(11) **EP 0 781 536 B1**
(45) Date de publication et mention de la délivrance du brevet: **08.11.2000**
(21) Numéro de dépôt: 96420364.0
(22) Date de dépôt: 26.12.1996
(51) Int. Cl.: A61F 5/058

(54) **Orthése de blocage claviculaire**
Immobilisierungsorthese für das Schlüsselbein
Clavicle immobilization orthesis

(30) Priorité: 28.12.1995 FR 9515849
(43) Date de publication de la demande: 02.07.1997
(73) Titulaire: RICHARD FRERES S.A., 42530 Saint Genest Lerpt (FR)
(72) Inventeur: Richard, Dominique, 42160 Bonson (FR)
(74) Mandataire: Perrier, Jean-Pierre

(56) Documents cités:
- US-A- 3 141 456
- US-A- 3 718 137
- US-A- 3 897 776
- US-A- 4 570 619
- US-A- 4 589 406

## Description

L'invention est relative à une orthèse de blocage claviculaire.

Les orthèses de blocage claviculaire comprennent en général des sangles formant un "8" dont les deux boucles se croisent dans le dos et passent chacune sur l'épaule et plus précisément sur les clavicules, sur le thorax, avant de revenir sur le dos en passant sous les bras.

Il en est ainsi de l'orthèse décrite dans US-A-4570619.

L'invention concerne plus particulièrement les orthèses dans lesquelles, comme décrit dans US-A-3718137, US-A-3897776 et US-A-4589406, les extrémités supérieures et postérieures des sangles claviculaires sont liées l'une à l'autre en formant un "V" dont la pointe est soit fixée sur un coussin dorsal, comme dans US-A-4589406 soit fixée sur une sangle dorsale qui, comme dans US-A-3718137, et US-A-3897776, est reliée à un coussin dorsal ou à un anneau de maintien de deux autres boucles pour l'accrochage des extrémités inférieures et postérieures des sangles claviculaires. Dans ces trois documents, la sangle dorsale est très courte, de sorte, que même si elle est réglable, elle maintient la zone comportant les boucles à proximité de la pointe du "V". Lors du serrage de ces sangles, la tension longitudinale qui leur est communiquée tend à rapprocher du cou leur pointe supérieure en "V" et ainsi à amener le coussin ou l'anneau sur les omoplates. Les mouvements du porteur conduisent aux mêmes effets.

Cela n'a pas d'incidence sur le maintien claviculaire, mais par contre génère un inconfort pour le porteur .

En effet, lorsque le porteur est en appui contre le dossier d'un siège ou lorsqu'il est couché sur le dos, ce qui est d'ailleurs la seule position qu'il peut occuper sans douleur, les boucles dorsales déposées sur les omoplates génèrent, malgré le coussin , une gêne qui, très rapidement, devient source de douleurs et d'inconfort.

L'inconfort résultant du mauvais positionnement du coussin et de la présence des boucles et de l'anneau est si important qu'il empêche le patient de dormir sur le dos, et dans certains cas, de rester allongé trop longtemps sur le dos.

L'inconfort est accru lorsque chaque boucle d'accrochage des extrémités inférieures des deux sangles claviculaires est reliée au coussin par un ruban traversant un anneau commun, car l'anneau et les trois boucles forment des surépaisseurs qui sont très perceptibles lorsqu'elles s'appuient sur les omoplates.

Un autre inconvénient de la fixation des sangles sur un anneau central lié au coussin dorsal est qu'elles favorisent l'orientation des extrémités des sangles sensiblement à 120° les unes des autres, c'est à dire procurent une répartition des sangles favorisant le déplacement du coussin sur les omoplates.

La présente invention a pour but de remédier à ces inconvénients en fournissant une orthèse de blocage claviculaire procurant un bon positionnement du coussin et répartissant les efforts s'exerçant sur le coussin, de manière que ce dernier ne tende pas à se déplacer et à venir sur les omoplates, tout en supprimant les points durs perceptibles en position couchée.

A cet effet, dans l'orthèse selon l'invention, la sangle dorsale a une longueur permettant, lorsque la pointe du "V" est disposée au niveau du cou, d'amener le coussin dans l'intervalle entre les omoplates, tandis que chacune des boucles portées par le coussin pour l'accrochage des parties postérieures des sangles claviculaires est fixée à l'extrémité d'un ruban sensiblement horizontal cousu sur ce coussin, pour procurer un accrochage maintenant sensiblement horizontalement ces parties postérieures.

Grâce à cet agencement l'orthèse est parfaitement calée, en translation verticale vers le haut, d'une part, par appui de la pointe en "V" des extrémités supérieures des sangles claviculaires contre la face postérieure du cou, et, d'autre part, par le bandeau horizontal qui, formé par les parties postérieures de ces sangles, s'appuie contre le dos en formant organe de calage.

Avantageusement, les angles supérieurs du coussin dorsal sont biseautés pour former deux butées aptes à venir en appui contre le bord inférieur des omoplates.

Ainsi, quand les parties inférieures des omoplates du porteur son saillantes, elles coopèrent avec les faces de butée du coussin pour assurer un calage parfait du coussin.

D'autres caractéristiques et avantages ressortirons de la description qui suit en référence au dessin schématique annexé représentant, à titre d'exemple, une forme d'exécution de cette orthèse de blocage claviculaire.

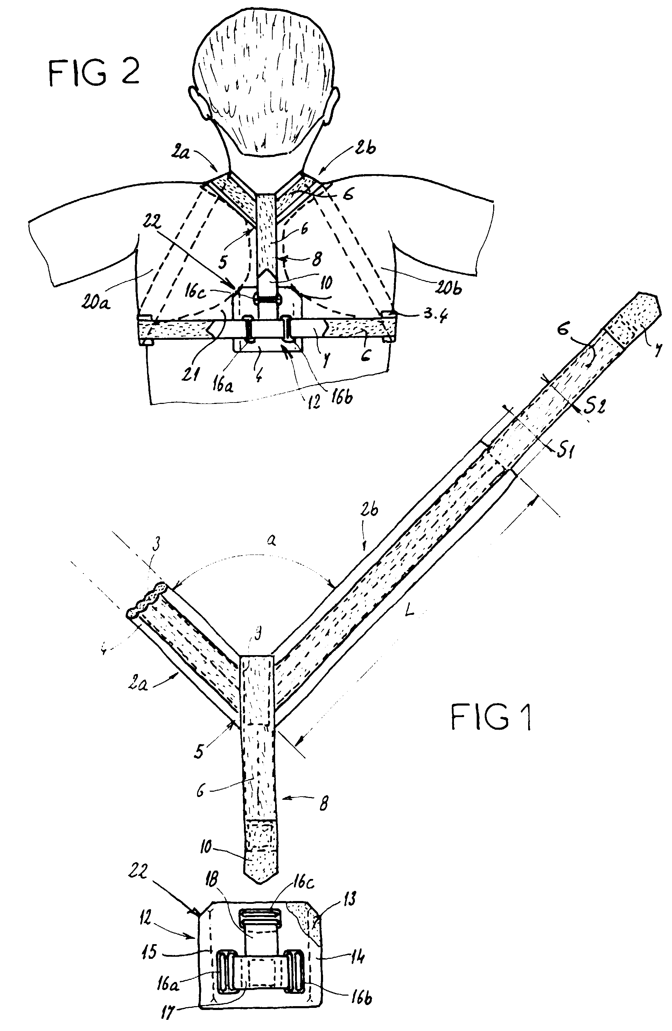

Figure 1 est une vue partielle de face en élévation montrant les éléments de l'orthèse, Figure 2 est une vue par l'arrière de l'orthèse lorsqu'elle est mise en place sur un patient.

Dans ce dessin, les références numériques 2a et 2b désignent, de manière générale, les deux sangles claviculaires qui, comme montré à la figure 1, forment un "V" dont l'angle d'ouverture a est de 90°, dans la forme d'exécution représentée, mais peut présenter une valeur différente. Chacune des sangles est composée d'un matelas 3, en mousse synthétique, disposé dans une enveloppe 4 en coton. Le matelas et l'enveloppe s'étendent, depuis la pointe 5 du "V", sur la longueur L, qui est définie selon la taille de l'orthèse et de manière que ce matelas puissent envelopper le flanc du patient.

Chaque sangle 2a, 2b comprend également un ruban en velours à bouclettes 6 qui est fixé sur son dos, pour en pas venir en contact avec le corps du porteur et qui, partant de la pointe 5, se prolonge au-delà du matelas pour former sangle d'accrochage. A son extrémité, le ruban 6 est muni d'un tronçon 7 de ruban à crochets, dont les crochets sont tournés du même côté que les bouclettes. La partie du ruban 6 prolongeant le matelas est doublée sur sa face venant contre le dos du porteur, par une nappe en coton cousue sur lui.

La sangle dorsale 8 est également constituée par un ruban 6 de velours à bouclettes doublé, dans sa partie venant contre le dos du porteur, par une nappe en coton. L'extrémité supérieure de la sangle dorsale 8 est fixée, par des coutures 9, sur la pointe 5 des sangles claviculaires et son extrémité inférieure est munie d'un tronçon 10 de ruban à crochets, dont les crochets sont tournés du même côté que les bouclettes, c'est-à-dire à l'opposé de sa partie venant en contact avec le dos de l'utilisateur.

la figure 1 met en évidence que le matelas 3 de chacune des sangles latérales 2a ou 2b ont une largeur S1 qui est supérieure à la largeur S2 du ruban 6, afin que les bords de ce ruban 6 ne puissent pas blesser le patient dans sa partie où la sangle s'appuie sur la peau.

Par ailleurs, pour obtenir une bonne tenue, les rubans 6 des sangles claviculaires et de la sangle dorsale 8 ont une largeur S2 qui est au moins de 30 mm, et par exemple de 35 mm, pour mieux répartir l'effort de plaquage sur la peau.

Le coussin 12, associé aux sangles, est composé d'un matelas 13, par exemple en feutre, disposé dans une enveloppe à coton 14 à laquelle il est lié par des coutures 15. Ce coussin supporte trois boucles doubles, à savoir deux boucles latérales 16a, 16b pour l'accrochage des extrémités des sangles claviculaires 2a, 2b, et une boucle supérieure 16c, pour l'accrochage de la sangle dorsale 8. Les deux boucles latérales 16a, 16b sont fixées dans les extrémités en forme d'anneau d'un ruban 17, qui est cousu, sensiblement horizontalement sur le coussin 12, tandis que la boucle 16c est fixée dans l'extrémité en forme d'anneau d'un ruban 18, sensiblement vertical et également cousu sur le coussin 12.

Lorsque l'orthèse est mise en place sur le patient et, comme montré à la figure 2, la pointe 5 est disposée à l'arrière du cou et plus précisément contre la face arrière de celui-ci, et les sangles 2a, 2b passent sur les clavicules, sur le thorax, puis sur les flancs en passant au-dessous des bras, avant de revenir en arrière.

Avant l'accrochage des sangles claviculaires 2a, 2b, l'extrémité de la sangle dorsale 8 est introduite dans la boucle 16c du coussin 12 et est réglée en longueur, de manière que ce coussin prenne appui sur le dos, dans l'intervalle 21 entre les deux omoplates 20a, 20b, comme montré à la figure 2, puis les extrémités des sangles latérales sont à leur tour engagées dans les boucles 16a et 16b.

La fixation de la partie postérieure de chacune des sangles 2a, 2b et 8 est assurée, après passage dans les boucles correspondantes, par engagement des crochets de leurs tronçons extrêmes 7 et 10 dans les bouclettes des rubans 6.

L'extrémité de chaque sangle repliée dans une boucle double, est pliée autour de la barrette centrale de la boucle correspondante 16a, 16b, 16c, et passe sous la barrette extrême pour former un embarrage assurant une liaison indéserrable.

La figure 2 montre bien que, grâce à la disposition horizontale des parties postérieures des sangles claviculaires, l'effort de réaction transmis au coussin 12 par le serrage de ces sangles 2a, 2b, n'induit pas de composantes verticales, ce qui, compte tenu du parfait réglage de la sangle dorsale 8, permet de maintenir ce coussin 12 dans la position qui lui est assignée au départ. Dans cette position, le coussin 12, de plus faible épaisseur que les coussins traditionnels, s'insèrent parfaitement entre les omoplates, ce qui lui permet, lorsque le patient est allongé sur le dos, de s'effacer avec les boucles 16a à 16 c, sans créer aucune gêne.

Avantageusement, les angles supérieurs du coussin 8 sont chanfreinés en 22 pour former des butées qui, en cas de faible déplacement vertical du coussin, par exemple par les mouvements du porteur, viennent en appui contre le bord inférieur des omoplates et complètent le calage en translation assuré par l'appui contre le cou de la pointe 5 du "V" des sangles.

## Revendications

1. Orthèse de blocage claviculaire comprenant une sangle dorsale, centrale et verticale (8), deux sangles claviculaires (2a, 2b), ayant des extrémités supérieures formant un "V" dont la pointe arrière (5) est fixée à l'extrémité supérieure de la sangle dorsale (8), et un coussin dorsal (12) muni de boucles (16a, 16b, 16c) pour l'accrochage, respectivement, des extrémités postérieures des deux sangles claviculaires (2a, 2b) et de la sangle dorsale (8) et pour le réglage en longueur de ces sangles, **caractérisée en ce que** la sangle dorsale (8) a une longueur permettant, lorsque la pointe (5) du "V" est disposée au niveau du cou, d'amener le coussin (12) dans l'intervalle entre les omoplates, tandis que chacune des boucles (16a, 16b), portées par le coussin (12) pour l'accrochage des parties postérieures des sangles claviculaires (2a, 2b), est fixée à l'une des extrémités d'un ruban (17) sensiblement horizontal, cousu sur ce coussin, pour procurer un accrochage maintenant sensiblement horizontalement ces parties postérieures.

2. Orthèse de blocage selon la revendication 1, **caractérisée en ce que** les angles supérieurs du coussin dorsal (12) sont biseautées pour former deux butées (22), aptes à venir en appui contre le bord inférieur des omoplates.

3. Orthèse de blocage claviculaire selon revendication 1, **caractérisée en ce que** chacune des boucles (16a, 16b) pour les sangles claviculaires (2a, 2b) est double et comporte deux barrettes pour former un embarrage avec l'extrémité repliée de la sangle correspondante.

4. Orthèse de blocage selon l'une quelconque des revendications 1 à 3, **caractérisée en ce que** le ruban des sangles claviculaires (2a, 2b) et de la sangle dorsale (8) a une largeur au moins égale à 30 mm.

5. Orthèse de blocage selon l'une quelconque des revendications 1 à 4, **caractérisée en ce que** la sangle dorsale (8) est composée d'une nappe en coton doublée, sur sa face externe et sur toute sa longueur, par un ruban (6) de velours à bouclettes et est munie, à son extrémité libre, d'un tronçon (10) de velours à crochets dont les crochets sont tournées du même côté que les bouclettes du ruban (6).

6. Orthèse de blocage selon l'une quelconque des revendications 1 à 5, **caractérisée en ce que** le ruban (6) de chaque sangle claviculaire (2a, 2b) est un ruban à bouclettes et son extrémité libre est munie d'un tronçon (7) de ruban à crochets dont les crochets sont tournés du même côté que les bouclettes du ruban (6).

## Patentansprüche

1. Schlüsselbeinimmobilisierungsorthese, umfassend einen zentralen und vertikalen Rückengurt (8), zwei Schlüsselbeingurte (2a, 2b) mit oberen Enden, die ein "V" bilden, dessen hintere Spitze (5) am oberen Ende des Rückengurts (8) befestigt ist, sowie ein Rückenkissen (12), das mit Schlaufen (16a, 16b, 16c) zum Befestigen der hinteren Enden der zwei Schlüsselbeingurte (2a, 2b) bzw. des Rückengurts (8) und zum Einstellen dieser Gurte in der Länge ausgestattet ist, **dadurch gekennzeichnet**, daß der Rückengurt (8) eine Länge aufweist, die es ermöglicht, das Kissen (12) in den Zwischenraum zwischen den Schulterblättern zu bringen, wenn die Spitze (5) des "V" im Bereich des Halses angeordnet ist, wohingegen jede der vom Kissen (12) getragenen Schlaufen (16a, 16b) zur Befestigung der hinteren Bereiche der Schlüsselbeingurte (2a, 2b) an einem der Enden eines im wesentlichen horizontalen, auf dieses Kissen genähten Bands (17) befestigt ist, um eine Befestigung zu bewirken, die diese hinteren Bereiche im wesentlichen horizontal hält.

2. Immobilisierungsorthese nach Anspruch 1, **dadurch gekennzeichnet**, daß die oberen Ecken des Rückenkissens (12) abgeschrägt sind, um zwei Anschläge (22) zu bilden, die dazu ausgelegt sind, in Anlage an den unteren Rand der Schulterblätter zu gelangen.

3. Schlüsselbeinimmobilisierungsorthese nach Anspruch 1, **dadurch gekennzeichnet**, daß jede der Schlaufen (16a, 16b) für die Schlüsselbeingurte (2a, 2b) doppelt ist und zwei Stangen umfaßt, um mit dem umgelenkten Ende des zugeordneten Gurts eine Spannvorrichtung zu bilden.

4. Immobilisierungsorthese nach einem der Ansprüche 1 bis 3, **dadurch gekennzeichnet**, daß das Band der Schlüsselbeingurte (2a, 2b) und des Rückengurts (8) eine Breite von wenigstens 30 mm aufweist.

5. Immobilisierungsorthese nach einem der Ansprüche 1 bis 4, **dadurch gekennzeichnet,** daß der Rückengurt (8) durch eine Baumwollschicht gebildet ist, die an ihrer Außenfläche und über ihre gesamte Länge mit einem Veloursband (6) mit Schlaufen ausgekleidet ist, und an seinem freien Ende mit einem Veloursabschnitt (10) mit Haken ausgestattet ist, dessen Haken auf der gleichen Seite wie die Schlaufen des Bands (6) angeordnet sind.

6. Immobilisierungsorthese nach einem der Ansprüche 1 bis 5, **dadurch gekennzeichnet**, daß das Band (6) jedes Schlüsselbeingurts (2a, 2b) ein Band mit Schlaufen ist, und daß sein freies Ende mit einem Bandabschnitt (7) mit Haken ausgestattet ist, dessen Haken auf der gleichen Seite wie die Schlaufen des Bands (6) angeordnet sind.

## Claims

1. A clavicle immobilisation orthesis comprising a dorsal, central and vertical strap (8), two clavicular straps (2a, 2b) having upper ends forming a "V", of which the point (5) is fixed at the rear to the upper end of the dorsal strap (8), and a dorsal pad (12) provided with buckles (16a, 16b, 16c) for fastening the posterior ends of the two clavicular straps (2a, 2b) and the dorsal strap (8) respectively and for adjusting the length of these straps,
characterised in that the dorsal strap (8) is of a length which, when the point (5) of the "V" is arranged at the level of the neck, allows the pad (12) to be moved into the gap between the scapulae, while each of the buckles (16a, 16b), carried by the pad (12) for fastening the posterior portions of the clavicular straps (2a, 2b), is fixed to one of the ends of a substantially horizontal band (17) sewn to said pad in order to provide a fastening which holds these posterior portions substantially horizontally.

2. A clavicle immobilisation orthesis according to claim 1,
characterised in that the upper corners of the dorsal pad (12) are chamfered to form two stops (22) suitable for resting against the lower edges of the scapulae.

3. A clavicle immobilisation orthesis according to claim 1,
characterised in that each of the buckles (16a, 16b) for the clavicular straps (2a, 2b) is double and comprises two bars to form a clasp with the folded-hack end of the corresponding strap.

4. A clavicle immobilisation orthesis according to any one of claims 1 to 3, characterised in that the band of the clavicular straps (2a, 2b) and the dorsal strap (8) has a length at least equal to 30 mm.

5. A clavicle immobilisation orthesis according to any one of claims 1 to 4, characterised in that the dorsal strap (8) comprises a layer of cotton lined, on its outer surface and over its entire length, with a looped velvet band (6) and is provided at its free end with a hooked velvet piece (10), the hooks facing towards the same side as the loops of the band (6).

6. A clavicle immobilisation orthesis according to any one of claims 1 to 5, characterised in that the band (6) of each clavicular strap (2a, 2b) is a looped band and its free end is provided with a hooked band piece (7), the hooks of which face towards the same side as the loops of the band (6).
